# EUROPÄISCHE PATENTSCHRIFT

(11) **EP 1 014 936 B1**
(45) Veröffentlichungstag und Bekanntmachung des Hinweises auf die Patenterteilung: **27.08.2003**
(21) Anmeldenummer: 98948955.4
(22) Anmeldetag: 10.09.1998
(51) Int. Cl.: A61K 7/48, A61K 7/06

(54) **EXTRAKT UND KOSMETISCHE ZUBEREITUNG**
EXTRACT AND COSMETIC PREPARATION
EXTRAIT ET PREPARATION COSMETIQUE

(30) Priorität: 19.09.1997 DE 19741397
(43) Veröffentlichungstag der Anmeldung: 05.07.2000
(73) Patentinhaber: Henkel Kommanditgesellschaft auf Aktien, 40191 Düsseldorf (DE)
(72) Erfinder: HÖFFKES, Horst, D-40595 Düsseldorf (DE); BERGMANN, Bettina, D-47269 Duisburg (DE); MÖLLER, Hinrich, D-40789 Monheim (DE); KLEEN, Astrid, D-40627 Düsseldorf (DE)
(86) Internationale Anmeldenummer: EP9805775
(87) Internationale Veröffentlichungsnummer: WO99015145

(56) Entgegenhaltungen:
- FR-A- 2 473 310
- PATENT ABSTRACTS OF JAPAN vol. 096, no. 005, 31. Mai 1996 & JP 08 026960 A (TSUNEO NANBA;OTHERS: 01), 30. Januar 1996
- PATENT ABSTRACTS OF JAPAN vol. 096, no. 009, 30. September 1996 & JP 08 113515 A (NANBA TSUNEO;MIKIMOTO PHARMACEUT CO LTD), 7. Mai 1996
- DATABASE WPI Section Ch, Week 9614 Derwent Publications Ltd., London, GB; Class B04, AN 96-136155 XP002095660 & JP 08 026960 A (MIKIMOTO SEIYAKU KK) , 30. Januar 1996
- DATABASE WPI Section Ch, Week 9551 Derwent Publications Ltd., London, GB; Class B04, AN 95-400910 XP002095661 & JP 07 278003 A (NARISU KESHOHIN KK) , 24. Oktober 1995
- DATABASE WPI Section Ch, Week 9344 Derwent Publications Ltd., London, GB; Class B04, AN 93-348356 XP002095662 & JP 05 255100 A (LOTTE CO LTD) , 5. Oktober 1993
- DATABASE WPI Section Ch, Week 9612 Derwent Publications Ltd., London, GB; Class B04, AN 96-112640 XP002095663 & JP 08 012585 A (LOTTE CO LTD) , 16. Januar 1996
- DATABASE WPI Section Ch, Week 9534 Derwent Publications Ltd., London, GB; Class B04, AN 95-261213 XP002095664 & JP 07 165599 A (SOYAKU GIJUTSU KENKYUSHO KK), 27. Juni 1995
- DATABASE WPI Section Ch, Week 9330 Derwent Publications Ltd., London, GB; Class B04, AN 93-239906 XP002095665 & JP 05 163128 A (POLA CHEM IND INC) , 29. Juni 1993

## Beschreibung

Die Erfindung betrifft spezielle Henna-Extrakte, ihre Verwendung als kosmetische Wirkstoffe sowie entsprechende Zubereitungen zur Behandlung von Haut und Haaren.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen. Färben und Verformen der Haare mit Wellmitteln, Tönungsmitteln und Stylingpräparaten. Während früher bei der Formulierung dieser Mittel vor allem diese Produkteigenschaften im Zentrum standen, wird nunmehr in immer größerem Maße angestrebt, mit diesem Mittel gleichzeitig auch eine konditionierende und pflegende Wirkung, ja eine Regeneration geschädigter Haare oder sogar einen präventiven Schutz vor Schädigungen, zu erzielen.

Dem Fachmann sind eine Reihe pflegender und konditionierender Wirkstoffe bekannt: jedoch besteht weiterhin ein Bedürfnis nach neuen Wirkstoffen. Besonderes Interesse gilt dabei neuen Wirkstoffen aus natürlichen, insbesondere pflanzlichen Quellen, die beim Verbraucher auf besonders hohe Akzeptanz stoßen.

Bereits seit dem Altertum ist die Verwendung von Henna zu Färbezwecken, insbesondere auch zur Färbung von menschlichen Haaren, bekannt. Dabei wurde auch schon frühzeitig beobachtet, daß durch das Auftragen von Henna-Zubereitungen (in der Regel als wäßrige Aufschlämmung in Form eine Färbebreies) nicht nur eine Anfärbung der Haare, sondern auch ein verbesserter Griff und ein erhöhter Glanz erzielt werden.

Aufgrund der intensiven Färbungen der bekannten Henna-Konfektionierungsformen blieb die Ausnutzung dieser pflegenden Wirkung lange Zeit auf das Haarfarbengebiet beschränkt. Zur Überwindung dieser Einschränkung gab es aber in jüngster Zeit eine Reihe von Ansätzen. Insbesondere wurde versucht, möglichst farblose Extrakte bereitzustellen, die gleichwohl die pflegende Wirkung der entsprechenden Hennapflanze aufweisen. In diesem Zusammenhang wird beispielhaft auf den Artikel von D. Kenney in Cosmetics & Toiletries, Vol 96 (Juni 1980), S. 44-51, verwiesen.

Die bekannten Henna-Extrakte können die gestellten Anforderungen aber bei weitem noch nicht zufriedenstellend erfüllen. Diese wäßrigen bzw. wäßrig-alkoholischen Extrakte weisen noch eine ausgeprägte Färbung auf. Dies kann einerseits zu unerwünschten Anfärbungen am Haar führen, macht aber insbesondere die farbliche Einstellung der diese Extrakte enthaltenden Endprodukt schwierig oder, wie im Falle farbloser, transparenter Produkte, praktisch unmöglich. Ein entscheidender Nachteil dieser Extrakte ist aber, daß die konditionierende und pflegende Wirkung sehr viel geringer ist als bei Verwendung der Henna-Pflanze selbst.

Es wurde nun überraschenderweise gefunden, daß durch Extraktion spezieller Teile bestimmter Typen von Henna-Pflanzen mit ausgewählten Lösungsmitteln farblose oder lediglich schwach gelblich gefärbte Extrakte erhalten werden, die im Vergleich zu bekannten Henna-Extrakten bei gleicher Einsatzmenge eine signifikant höhere Pflegeund Konditionierwirkung auf Haut und Haar entfalten.

Ein erster Gegenstand der Erfindung sind daher Extrakte, erhältlich durch Extraktion von Blättern, Blattstielen und/oder Zweigen von Pflanzen, ausgewähtt aus Cassia auriculata oder Lawsonia inermis mit einem organischen Lösungsmittel, das bei 20 °C eine Dielektrizitätskonstante von 2,5 oder kleiner aufweist.

Extrakte von Lawsonia inermis zeichnen sich durch eine besonders starke Wirkung aus.

Wenngleich sich sowohl Blätter als auch Blattstiele und Zweige der genannten Pflanzen als geeignete Ausgangsmaterialien erwiesen haben, werden die Extrakte doch bevorzugt aus den gemahlenen Blättern dieser Pflanzen hergestellt.

Um zu den erfindungsgemäßen Extrakten zu gelangen, dürften die für die Extraktion verwendeten Lösungsmittel nur eine geringe Polarität aufweisen. Geeignet sind alle Lösungsmittel mit einer Dielektrizitätskonstanten von 2,5 und kleiner bei 20 °C. Die Dielektrizitätskonstanten der Lösungsmittel kann der Fachmann den bekannten einschlägigen Nachschlagewerken, beispielsweise dem "CRC Handbook of Chemistry and Physics", entnehmen.

Erfindungsgemäß bevorzugt sind solche Lösungsmittel, die bei 20 °C in Wasser zu weniger als 1 Gew.-% in Wasser löslich sind.

Ganz besonders bevorzugte Lösungsmittel im Rahmen der erfindungsgemäßen Lehre sind Cyclohexan, Tetrachlormethan, Benzol, n-Hexan und n-Pentan. Unter Verwendung von Cyclohexan als Lösungsmittel hergestellte Extrakte haben sich als ganz besonders wirksam erwiesen.

Die Extraktion erfolgt üblicherweise unter Normaldruck, Gemäß einer speziellen Ausführungsform der Erfindung kann der Extrakt aber auch unter Verwendung von Lösungsmitteln im überkritischen Zustand hergestellt werden. In diesem Fall kann die Verwendung von n-Pentan als Lösungsmittel bevorzugt sein.

Gemäß einer bevorzugten Ausführungsform der Erfindung ist der Extrakt frei von färbenden Anteilen. Als "frei von färbenden Anteilen" wird im Rahmen der erfindungsgemäßen Lehre ein Extrakt angesehen, wenn er die folgenden Bedinungen erfüllt:
- keine mit bloßem Auge erkennbare oder lediglich geringfügig gelbliche Färbung des Extraktes selbst
- keine mit bloßem Auge erkennbare Anfärbung von Haut oder Haaren beim Auftragen des Extraktes
- keine Wechselwirkung mit anderen im Produkt enthaltenen Farbstoffen, die eine mit bloßem Auge erkennbare Farbveränderung bewirkt.

Das erfindungsgemäß bei der Extraktion erhaltene Wirkstoff/Lösungsmittel-Gemisch wird durch Entfernen, z.B. Verdampfen, der überwiegenden Menge des Lösungsmittels stark eingeengt, so daß der erhaltene Extrakt vorzugsweise weniger als 5 Gew.-% an ursprünglichem Lösungsmittel, bezogen auf den gesamten Extrakt, enthält. Besonders bevorzugt sind solche Extrakte, die keine nachweisbaren Mengen an ursprünglichem Lösungsmittel mehr enthalten.

Ein weiterer Gegenstand der Erfindung ist die Verwendung der oben bezeichneten Extrakte als kosmetischer Wirkstoff.

Ein dritter Gegenstand der Erfindung sind schließlich Zubereitungen zur Behandlung menschlicher Haut oder menschlicher Haare, die einen der oben bezeichneten Extrakte enthalten.

Die Art des Haarbehandlungsmittels unterliegt keinen prinzipiellen Beschränkungen. Es kann sich beispielsweise um reinigende Mittel wie Shampoos, pflegende Mittel wie Haarkuren und Haarspülungen, festigende Mitteln wie Haarfestiger, Haarsprays und Fönwellen, dauerhafte Verformungsmittel wie Dauerwell- und -fixiermittel. farbverändernde Mittel wie Blondiermittel, Oxidationsfärbemittel und Tönungsmittel auf Basis direktziehender Farbstoffe, Haarwässer und Haarspitzenfluids handeln. Entsprechend können die Zubereitungen als Lösungen, Emulsionen, Gele, Cremes, Aerosole oder Lotionen formuliert werden.

Haarpflegemittel einerseits und Haarfärbemittel andererseits sind im Rahmen der erfindungsgemäßen Lehre bevorzugte Haarbehandlungsmittel.

Die erfindungsgemäßen Zubereitungen enthalten als zwingende Komponente einen Extrakt gemäß Anspruch 1. Auf die bevorzugten Ausführungsformen hinsichtlich dieser Komponente wird auf das oben gesagte verwiesen.

Gemäß einer ersten Ausführungsform bestehen die erfindungsgemäßen Zubereitungen ausschließlich aus diesem Henna-Extrakt oder enthalten als weitere Komponenten lediglich Konservierungsmittel und/oder Parfümöle in Mengen von jeweils unter 1 Gew.-%, bezogen auf die gesamte Zubereitung.

Gemäß einer zweiten Ausführungsform enthalten die erfindungsgemäßen Zubereitungen diesen Extrakt in Mengen von 0.05 - 99, insbesondere 0.1 - 10, Gew.-%, bezogen zum einen auf den Aktivsubstanzgehalt des Extraktes und zum anderen auf die gesamte Zubereitung.

Die erfindungsgemäßen Haarbehandlungsmittel enthalten weiterhin bevorzugt mindestens ein Tensid. Es können sowohl anionische als auch nichtionische, kationische, zwitterionische und ampholytischer Tenside eingesetzt werden, wobei die Verwendung mindestens eines anionischen Tensids einerseits sowie mindestens eines kationischen oder nichtionogenen Tensids andererseits bevorzugt sein können.

Erfindungsgemäß verwendbare Tenside sind beispielsweise:
- anionische Tenside wie insbesondere Seifen von Fettsäuren, Alkylsulfate. Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethyleruppen,
- nichtionogene Tenside wie insbesondere Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe, C₁₂-C₂₂-Fettsäuremono- und -diester von Anlagerungsprodukten von 1 bis 30 Mol Ethylenoxid an Glycerin. C₈-C₂₂-Alkylmono- und -oligoglycoside und deren ethoxylierte Analoga sowie Anlagerungsprodukte von 5 bis 60 Mol Ethylenoxid an Rizinusöl und gehärtetes Rizinusöl,
- zwitterionische Tenside. insbesondere die sogenannten Betaine wie die N-Alkyl-N.N-dimethylammonium-glycinate, beispielsweise das Kokosalkyl-dimethylammonium-glycinat. N-Acyl-aminopropyl-N.N-dimethylammoniumglycinate, beispielsweise das Kokosacylaminopropyldime-thylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.
- ampholytische Tenside wie N-Alkylglycine. N-Alkylpropionsäuren. N-Alkylaminobuttersäuren, N-Alkyliminodipropionsäuren. N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe,
- kationische Tenside vom Typ der quartären Ammoniumverbindungen, z.B.Ammoniumhalogenide. insbesondere Chloride und Bromide. wie Alkyltrimethylammoniumchloride. Dialkyldimethylammoniumchloride und Trialkylmethylammoniumchloride, z. B. Cetyltrimethylammoniumchlorid. Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, Behenyltrimethylammoniummethosulfat sowie die unter den INCI-Bezeichnungen Quaternium-27 und Quaternium-83 bekannten Imidazolium-Verbindungen. Ebenfalls als kationische Tenside einsetzbar sind Alkylamidoamine wie das unter der Bezeichnung Tegoamid® S 18 im Handel erhältliche Stearamidopropyldimethylamin und sogenannte Esterquats wie das unter der Bezeichnung Armocare® VGH-70 im Handel erhältliche N,N-Bis(2-Palmitoyloxyethyl)dimethylammoniumchlorid.

Je nach Art des Mittels und des Tensidtyps können die Tenside in den erfindungsgemäßen Mitteln in Mengen von insgesamt 0.5 bis 25 Gew.-%, bezogen auf das gesamte Mittel, enthalten sein; Mittel, die als sogenannte Konzentrate formuliert sind, können auch höhere Tensidgehalte aufweisen.

Weiterhin enthalten die erfindungsgemäßen Zubereitungen bevorzugt noch einen Fettstoff.

Bevorzugte Fettstoffe sind lineare und verzweigte, gesättigte und ungesättigte Fettalkohole oder natürliche Fettalkoholgemische mit 8 bis 22 Kohlenstoffatomen in der Alkylkette wie beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Palmitylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole sowie Fettalkoholschnitte, die durch Reduktion natürlich vorkommender Triglyceride wie Rindertalg, Palmöl, Erdnußöl. Rüböl, Baumwollsaatöl. Sojaöl, Sonnenblumenöl und Leinöl oder aus deren Umesterungsprodukten mit entsprechenden Alkoholen entstehenden Fettsäureestern erzeugt werden und somit ein Gemisch von unterschiedlichen Fettalkoholen darstellen. Die Fettalkohole werden üblicherweise in Mengen von 0.01 bis 15 Gew.-%, bevorzugt von 0.1 bis 10 Gew.-% und besonders bevorzugt von 0,3 bis 6 Gew.-%. bezogen auf die gesamte Zubereitung, eingesetzt. Für spezielle Zubereitungen kann die Menge an Fettstoff aber auch bis zu 90 Gew.-% betragen.

Ebenfalls als Fettstoffe eingesetzt werden können Monoester der Fettsäuren mit Alkoholen mit 6 bis 24 C-Atomen sowie Triglyceride natürlichen Ursprungs.

Weiterhin können die erfindungsgemäßen Haarbehandlungsmittel bevorzugt noch einen konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise
- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quatemierte Cellulose-Derivate.
- polymere Dimethyldiallylammoniumsalze und deren Copolymere mit Estern und Amiden von Acrylsäure und Methacrylsäure. Die unter den Bezeichnungen Merquat®100 (Poly(dimethyldiallylammoniumchlorid)) und Merquat®550 (Dimethyldiallylammoniumchlorid-Acrylamid-Copolymer) im Handel erhältlichen Produkte sind Beispiele fiir solche kationischen Polymere.
- Copolymere des Vinylpyrrolidons mit quaternierten Derivaten des Dialkylaminoacrylats- und -methacrylats. wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Besonders bevorzugt sind kationische Polymere der vier erstgenannten Gruppen.

Als konditionierende Wirkstoffe weiterhin geeignet sind Silikonöle, insbesondere Dialkyl- und Alkylarylsiloxane, wie beispielsweise Dimethylpolysiloxan und Methylphenylpolysiloxan, sowie deren alkoxylierte und quaternierte Analoga. Beispiele fiir solche Silikone sind die von Dow Corning unter den Bezeichnungen DC 190, DC 200, DC 344, DC 345 und DC 1401 vertriebenen Produkte sowie die Handelsprodukte Q2-7224 (Hersteller: Dow Coming; ein stabilisiertes Trimethylsilylamodimethicon). Dow Corning® 929 Emulsion (enthaltend ein hydroxyl-amino-modifiziertes Silicon. das auch als Amodimethicone bezeichnet wird), SM-2059 (Hersteller: General Electric). SLM-55067 (Hersteller: Wacker) sowie Abil®-Quat 3270 und 3272 (Hersteller: Th. Goldschmidt: diquatemäre Polydimethylsiloxane, Quaternium-80).

Ebenfalls einsetzbar als konditionierende Wirkstoffe sind Paraffinöle sowie pflanzliche Öle wie Jojobaöl, Sonnenblumenöl. Orangenöl, Mandelöl, Weizenkeimöl und Pfirsichkemöl.

Gleichfalls geeignete haarkonditionierende Verbindungen sind Phospholipide, beispielsweise Sojalecithin. Ei-Lecithin und Kephaline.

Erfindungsgemäße Zubereitungen, die Haarfärbe- oder -tönungsmittel darstellen, können enthalten mindestens ein Oxidationsfarbstoffvorprodukt vom Entwicklertyp, ggf. in Kombination mit Oxidationsfarbstoffvorprodukten vom Kupplertyp, und/oder einen direktziehenden Farbstoff.

Erfindungsgemäß bevorzugte Entwicklerkomponemen sind p-Phenylendiamin, p-Toluylendiamin, p-Aminophenol, 1-(2'-Hydroxyethyl)-2,5-diaminobenzol, N,N-Bis-(2-hydroxy-ethyl)-p-phenylendiarnin, 2-(2,5-Diaminophenoxy)-ethanol, 1-Phenyl-3-carboxyamido-4-arnino-pyrazolon-5. 4-Amino-3-methylphenol, 2-Methylamino-4-aminophenol. 2,4,5,6-Tetraaminopyrimidin, 2-Hydroxy-4,5.6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylaminomethyl-4-amino-phenol sowie 4,4'-Diaminodiphenylamin.

Erfindungsgemäß bevorzugte Kupplerkomponenten sind 1-Naphthol, Pyrogallol, 1.5-. 2,7- und 1,7-Dihydroxynaphthalin, o-Aminophenol, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2,4-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2,6-Bis-(2-hydroxyethylamino)-toluol, 2,4-Diaminophenoxyethanol, 2-Amino-4-hydroxyethylamino-anisol.

Üblicherweise werden Entwicklerkomponenten und Kupplerkomponenten in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten bevorzugt in einem Mol-Verhältnis von 1 : 0.5 bis 1 : 2 im Färbemittel enthalten sein können. Die Gesamtmenge an Oxidationsfarbstoffvorprodukten liegt in der Regel bei höchstens 20 Gew.-%. bezogen auf das gesamte Mittel.

Direktziehende Farbstoffe sind üblicherweise Nitrophenylendiamine. Nitroaminophenole, Anthrachinone oder Indophenole. Bevorzugte direktziehende Farbstoffe sind die unter den internationalen Bezeichnungen bzw. Handelsnamen HC Yellow 2, HC Yellow 4, HC Yellow 6, Basic Yellow 57. Disperse Orange 3, HC Red 3, HC Red BN. Basic Red 76, HC Blue 2, Disperse Blue 3. Basic Blue 99, HC Violet 1. Disperse Violet 1, Disperse Violet 4, Disperse Black 9, Basic Brown 16 und Basic Brown 17 bekannten Verbindungen sowie 4-Amino-2-nitrodiphenylamin-2'carbonsäure, 6-Nitro-1,2,3,4-tetrahydrochinoxalin, Hydroxyethyl-2-nitro-toluidin.

Pikraminsäure, 2-Amino-6-chloro-4-nitrophenol und 4-N-Ethyl-1.4-bis(2'hydroxyethylamino)-2-nitrobenzol-hydrochlorid. Die erfindungsgemäßen Mittel gemäß dieser Ausführungsform enthalten die direktziehenden Farbstoffe bevorzugt in einer Menge von 0.01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein. soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak. The Science of Hair Care. Kapitel 7 (Seiten 248-250: direktziehende Farbstoffe) sowie Kapitel 8, Seiten 264-267: Oxidationsfarbstoffvorprodukte), erschienen als Band 7 der Reihe "Dermatology" (Hrg.: Ch., Culnan und H. Maibach), Verlag Marcel Dekker Inc., New York. Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V.. Mannheim. Bezug genommen.

Der pH-Wert der erfindungsgemäßen Zubereitungen kann prinzipiell zwischen 2 - 11 liegen, wobei der Fachmann die bevorzugten pH-Bereiche fiir die unterschiedlichen Mittel kennt. Der pH-Wert der erfindungsgemäßen Mittel, sofern es sich um Haarpflegemittel handelt, liegt bevorzugt zwischen 2 und 7, wobei Werte von 3 bis 5 besonders bevorzugt sind. Zur Einstellung dieses pH-Wertes kann praktisch jede für kosmetische Zwecke verwendbare Säure verwendet werden. Üblicherweise werden Genußsäuren verwendet, Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt, Haarfärbemittel werden bevorzugt in einem pH-Bereich von etwa 7 bis 11 formuliert.

Weitere übliche Bestandteile der erfindungsgemäßen Zubereitungen können sein:
- Anionische, zwitterionische, amphotere und nichtionische Polymere wie beispielsweise Vinylacetat/Crotonsäure-Copolymere, Polydimethylsiloxane, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Acryl-amidopropyl-trimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenfalls derivatisierte Celluloseether.
- symmetrische und unsymmetrische, lineare und verzweigte Dialkylether mit insgesamt zwischen 12 bis 36 C-Atomen, insbesondere 12 bis 24 C-Atomen. beispielsweise Di-n-octylether, Di-n-decylether, Di-n-nonylether, Di-nundecylether und Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-noctylether, iso-Pentyl-n-octylether und 2-Methyl-pentyl-n-octylether,
- quarternierte Amine wie Methyl-1-alkylamidoethyl-2-alkylimidazolinium-methosulfat
- Entschäumer wie Silikone,
- Verdickungsmittel wie Agar-Agar, Guar-Gum, Alginate, Xanthan-Gum, Gelatine. Pektine, Hydroxyethylcellulose sowie Polyacrylamide und deren Copolymere,
- Strukturanten wie Maleinsäure. Mono-, Di- und Oligosaccharide,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe zum Anfärben des Mittels,
- Antischuppenwirkstoffe wie Piroctone Olamine, Zink Omadine und Climbazol,
- weitere Substanzen zur Einstellung des pH-Wertes,
- tierische und pflanzliche Proteinhydrolysate, insbesondere Elastin-, Kollagen-, Keratin-, Milcheiweiß-, Sojaprotein-, Mandelprotein- und Weizenproteinhydrolysate, sowie deren Fettsäurekondensationsprodukte und quaternierte Derivate,
- Vitamine und Vitaminvorstufen, wie Panthenol, dessen Derivate und Biotin,
- Pflanzen- und Honigextrakte, wie insbesondere Extrakte aus Eichenrinden, Brennessel, Hamarnelis, Hopfen, Kamille, Klettenwurzel, Schachtelhalm, Lindenblüten, Mandel, Aloe Vera, Kokosnuß, Mango, Aprikose, Limone, Weizen, Kiwi. Melone, Orange, Grapefruit, Salbei, Rosmarin, Birke, Wiesenschaumkraut, Quendel, Schafgarbe, Hauhechel, Meristem, Ginseng und Ingwerwurzel,
- Weitere Wirkstoffe wie Ceramide, Allantoin, Pyrrolidoncarbonsäuren, und Bisabolol,
- Lichtschutzmittel,
- Konsistenzgeber wie Zuckerester, Polyolester oder Polyolalkylether,
- Fette und Wachse, wie Walrat, Bienenwachs, Montanwachs und Paraffine,
- Fettsäurealkanolamide,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere,
- Perlelanzmittel wie Ethylenglykolmono- und -distearat sowie PEG-3-distearat,
- Komplexbildner wie EDTA, NTA, β-Alanindiessigsäure und Phosphonsäuren.
- Reduktionsmittel wie z.B. Thioglykolsäure und deren Derivate. Thiomilchsäure, Cysteamin, Thioäpfelsäure und α-Mercaptoethansulfonsäure,
- Oxidationsmittel wie Wasserstoffperoxid, Kaliumbromat und Natriumbromat,
- Treibmittel wie Propan-Butan-Gemische, N₂O, Dimethylether, CO₂, N₂ und Luft sowie
- Antioxidantien.

Bezüglich weiterer Bestandteile sowie Mengenbereiche für die einzelnen Inhaltsstoffe wird auf die dem Fachmann bekannten Handbücher, z.B. K. Schrader, Grundlagen und Rezepturen der Kosmetika, 2. Auflage, Hüthig Buch Verlag, Heidelberg, 1989. verwiesen.

### Beispiele

Alle Mengenangaben in den Beispielen sind, soweit nicht anders vermerkt, Gewichtsteile.

### 1. Herstellung der Extrakte

### 1.1 Aus färbenden Henna (Lawsonia inermis)-Blättern indischer Provenienz

200 g getrocknete pulverisierte Henna-Blätter wurden in 4 l Cyclohexan in einer Soxhlet-Apparatur 24 Stunden extrahiert. Die erhaltene Lösung wurde filtriert und im Rotavapor unter Wasserstrahlvakuum bis zur Lösungsmittelfreiheit eingeengt. Der Extrakt lag in Form eines schwach hellgelben Öles vor, Die Ausbeute betrug 7,3 %, bezogen auf das eingesetzte trockene Pflanzenmaterial.

### 1.2 Aus Henna neutral (Cassia auriculata)-Blätter- und -Rindenteilen

200 g getrocknete pulverisierte Blätter- und Rindenteile wurden in 4 1 Cyclohexan in einer Soxhlet-Apparatur 24 Stunden extrahiert. Die erhaltene Lösung wurde filtriert und im Rotavapor unter Wasserstrahlvakuum bis zur Lösungsmittelfreiheit eingeengt. Der Extrakt lag in Form eines farblosen Öles vor. Die Ausbeute betrug 6,5 %, bezogen auf das eingesetzte trockene Pflanzenmaterial

### 2. Verfahren zur Bestimmung der Naßkämmbarkeit

### 2.1 Gezielte Schädigung der Haarsträhnen

Trockene Haarsträhnen von ca. 2 g Gewicht (Fischbach und Miller, Typ 6923) wurden einmal mit 32 g Blondiermittel (Handelsprodukt Poly Blond Medium Aufheller) eine halbe Stunde lang blondiert. Nach dem Auswaschen der Blondiermischung wurden die Haarsträhnen unmittelbar ohne Zwischentrocknung einer Dauerwellbehandlung mit dem Marktprodukt Poly Lock Normal unterzogen. Die Einwirkdauern von Wellkomponente und Fixierkomponente betrugen dabei 30 bzw. 15 Minuten. Nach dem Ausspülen der Fixierkomponente wurden die Strähnen getrocknet und mindestens zwei Tage bei Umgebungsbedingungen konditioniert.

### 2.2 Bestimmung der Kämmbarkeit

Die gespülte Haarsträhne wurde vor der Bestimmung mit 0.2 ml einer 50%igen wäßrigen Lösung von Texapon® N25 (28%ige Lösung von Natriumlaurylethersulfat in Wasser) intensiv shampooniert und anschließend ausgespült. Danach wurde 1 g der zu prüfenden Zubereitung gleichmäßig in das Haar massiert. Auszuspülende Produkte wurden eine Minute auf dem Haar belassen und dann sorgfältig ausgespült. Nach dem Einmassieren (bei auf dem Haar verbleibenden Produkten) bzw. nach dem Ausspülen (bei abzuspülenden Produkten) wurde das Haar mit einem feinzinkigen Hartgummikamm gekämmt und der Kämmwiderstand subjektiv beurteilt. Vergleichszubereitungen wurden anschließend an der gleichen Strähne in gleicher Weise geprüft. Die Beurteilung wurde entsprechend einem Beurteilungssystem von 1 (= sehr gut) bis 5 (= sehr schlecht) abgegeben.

### 3. Anwendungsbeispiele

### 3.1 Haarpflegelotion/Haarglanzlotion (im Haar verbleibend)

| | B1a | B1b |
|---|---|---|
| Henna-Extrakt gem. Beispiel 1.1 | 0,5 | - |
| Henna-Extrakt gem. Beispiel 1.2 | - | 0,5 |
| Wasser | 50.0 | 50,0 |
| Ethanol | 49,5 | 49,5 |
| Naßkämmbarkeit | 1 | 3 |

Nach dem Trocknen war das Haar in beiden Fällen weich, glänzend und gut kämmbar. Es zeigte auch eine gegenüber dem ursprünglichen Zustand vor dem Auftragen des Mittels verringerte elektrostatische Aufladung.

### 3.2 Anionische Spülung

| | B2a | B2b | B2c | V2 |
|---|---|---|---|---|
| Talgfettalkohol | 8 | 8 | 8 | 8 |
| Texapon®N28¹ | 15 | 15 | 15 | 15 |
| Henna-Extrakt gem. Beispiel 1.1 | 1 | - | - | - |
| Henna-Extrakt gem. Beispiel 1.2 | - | - | 1 | 3 |
| Wasser | 76 | 76 | 74 | 77 |
| Beurteilung der Naßkämmbarkeit | 3 | 4 | 1 | 4-5 |

| | | | | |
|---|---|---|---|---|
| ¹ Laurylethersulfat-Natriumsalz (ca. 28 % Aktivsubstanz; INCI-Bezeichnung: Sodium Laureth Sulfat) (HENKEL) | | | | |

Damit entsprechen die Bewertungen der Spülung B2c denen, die üblicherweise für Zubereitungen mit quartären Ammoniumverbindungen vergeben werden.

### 3.3 Spülung auf Basis natürlicher Rohstoffe

| | B3 | V3 |
|---|---|---|
| Phospholipon®25 P² | 2 | 2 |
| Henna-Extrakt gem. Beispiel 1.1 | 2 | - |
| Guarkemmehl | 1 | 1 |
| Wasser | 97 | 95 |
| Beurteilung der Naßkämmbarkeit | 1 | 2 |

| | | |
|---|---|---|
| ² Soja-Lecithin, entölt (Rhone-Poulenc) | | |

### 3.4 Haartönung

| | B4 | V4 |
|---|---|---|
| Texapon®N 28 | 10,0 | 10,0 |
| C₁₂₋₁₈-Fettalkoholgemisch | 3,5 | 3,5 |
| Henna-Extrakt gem. Beispiel 1.1 | 2,0 | - |
| HC Blue No. 2 | 0,05 | 0,05 |
| Violett 1,4 D | 0,05 | 0,06 |
| HC Yellow 2 | 0,06 | 0,06 |
| 6-Nitro-1,2,3,4,-tetrahydrochinoxalin | 0,02 | 0,02 |
| Arianor Siennabraun | 0,05 | 0,05 |
| 2-Amino-2-methyl-propanol-1 | <----ad pH 8,0----> | |
| Wasser | <-----ad 100-----> | |
| | | |
| Farbton auf hellblonden Haarsträhnen | <---honigblond---> | |
| Beurteilung der Naßkämmbarkeit | 3 | 4-5 |

### 3.5 Cremeshampoo/Duschbad.

| | B5a | B5b | V5a | V5b |
|---|---|---|---|---|
| Kokosfettalkohol | 3,0 | 3,0 | 4,0 | 5,0 |
| Texapon®N 28 | 30,0 | 25,0 | 25,0 | 30,0 |
| Henna-Extrakt gem. Beispiel 1.1 | 2,0 | 1,0 | - | - |
| Natriumstearat | 3,0 | - | - | 3,0 |
| Wasser | 62,0 | 71,0 | 71,0 | 62,0 |
| Beurteilung der Naßkämmbarkeit | | 3 | 4-5 | |

Rezeptur B5a hinterließ nach dem Duschen einen deutlich spürbareren Pflegeeffekt als Rezeptur V5b

### 3.6 Hautpflegelotion

| | |
|---|---|
| Lanette®N³ | 8,0 |
| Cetiol®OE⁴ | 2,0 |
| Henna-Extrakt gem. Bsp. 1.1 | 2,0 |
| Eutanol®G⁵ | 4,0 |
| Konservierungsmittel | q.s. |
| Wasser | ad 100 |

| | |
|---|---|
| Die Lotion ist auf der Haut sehr gut verteilbar und gibt ihr ein angenehmes weiches Gefühl. ³ Cetylstearylalkohol-Fettalkoholsulfat-Gemisch 90:10 (INCI-Bezeichnung: Cetearyl Alcohol. Sodium Ceteareth Sulfate (HENKEL) | |
| ⁴ Dioctylether (INCI-Bezeichnung: Dicaprylether) (HENKEL) | |
| ⁵ 2-Octyldodecanol (INCI-Bezeichnung: Octyldodecanol) (HENKEL) | |

## Patentansprüche

1. Extrakt, erhältlich durch Extraktion von Blättern, Blattstielen und/oder Zweigen von Pflanzen, ausgewählt aus Lawsonia inermis und Cassia auriculata, mit einem organischen Lösungsmittel, das bei 20 °C eine Dielektrizitätskonstante von 2,5 oder kleiner aufweist.

2. Extrakt nach Anspruch 1, **dadurch gekennzeichnet, daß** die Pflanze Lawsonia inermis ist.

3. Extrakt nach einem der Ansprüche 1 oder 2, **dadurch gekennzeichnet, daß** der Extrakt durch Extraktion von gemahlenen Blättern erhalten wurde.

4. Extrakt nach einem der Ansprüche 1 bis 3, **dadurch gekennzeichnet, daß** das organische Lösungsmittel bei 20 °C zu weniger als 1 Gew.-% in Wasser löslich ist.

5. Extrakt nach einem der Ansprüche 1 bis 4, **dadurch gekennzeichnet, daß** das organische Lösungsmittel ausgewählt ist aus Cyclohexan, Tetrachlormethan, Benzol, n-Hexan und n-Pentan.

6. Extrakt nach einem der Ansprüche 1 bis 5, **dadurch gekennzeichnet, daß** das Lösungsmittel Cyclohexan ist.

7. Extrakt nach einem der Ansprüche 1 bis 5, **dadurch gekennzeichnet, daß** das Lösungsmittel n-Pentan ist.

8. Extrakt nach einem der Ansprüche 1 bis 7, **dadurch gekennzeichnet, daß** der Extrakt frei von färbenden Anteilen ist.

9. Verwendung eines Extraktes nach einem der Ansprüche 1 bis 8 als kosmetischer Wirkstoff.

10. Zubereitung zur Behandlung von menschlicher Haut oder menschlichen Haaren, **dadurch gekennzeichnet, daß** die Zubereitung einen Extrakt nach einem der Ansprüche 1 bis 8 enthält.

11. Zubereitung nach Anspruch 10, **dadurch gekennzeichnet, daß** es sich um ein Haarbehandlungsmittel, insbesondere ein Haarpflegemittel oder ein Haarfärbemittel, handelt.

12. Zubereitung nach Anspruch 10, **dadurch gekennzeichnet, daß** es sich um ein Hautbehandlungsmittel handelt.

13. Zubereitung nach einem der Ansprüche 10 bis 12, **dadurch gekennzeichnet, daß** sie weiterhin ein Tensid enthält.

14. Zubereitung nach einem der Ansprüche 10 bis 13, **dadurch gekennzeichnet, daß** sie weiterhin eine Fettstoff enthält

## Claims

1. An extract obtainable by extracting leaves, leaf stems and/or branches of plants selected from Lawsonia inermis and Cassia auriculata with an organic solvent which has a dielectric constant of 2.5 or lower at 20°C.

2. An extract as claimed in claim 1, **characterized in that** the plant is Lawsonia inermis.

3. An extract as claimed in claim 1 or 2, **characterized in that** the extract was obtained by extracting ground leaves.

4. An extract as claimed in any of claims 1 to 3, **characterized in that** the organic solvent has a solubility in water of less than 1% by weight at 20°C.

5. An extract as claimed in any of claims 1 to 4, **characterized in that** the organic solvent is selected from cyclohexane, tetrachloromethane, benzene, n-hexane and n-pentane.

6. An extract as claimed in any of claims 1 to 5, **characterized in that** the solvent is cyclohexane.

7. An extract as claimed in any of claims 1 to 5, **characterized in that** the solvent is n-pentane.

8. An extract as claimed in any of claims 1 to 7, **characterized in that** the extract is free from coloring components.

9. The use of the extract claimed in any of claims 1 to 8 as a cosmetic agent.

10. A preparation for treating human skin or human hair, **characterized in that** it contains the extract claimed in any of claims 1 to 8.

11. A preparation as claimed in claim 10, **characterized in that** it is a hair treatment preparation, more particularly a hair-care preparation or a hair colorant.

12. A preparation as claimed in claim 10, **characterized in that** it is a skin-care preparation.

13. A preparation as claimed in any of claims 10 to 12, **characterized in that** it additionally contains a surfactant.

14. A preparation as claimed in any of claims 10 to 13, **characterized in that** it additionally contains a fetty compaund.

## Revendications

1. Extrait pouvant être obtenu par extraction de feuilles, de pétioles et/ou de rameaux de plantes choisies parmi Lawsonia inermis et Cassia auriculata, à l'aide d'une solvant organique qui présente une constante diélectrique à 20°C de 2,5 ou moins.

2. Extrait selon la revendication 1, **caractérisé en ce que** la plante est Lawsonia inermis.

3. Extrait selon la revendication 1 ou 2, **caractérisé en ce que** l'extrait est obtenu par extraction de feuilles broyées.

4. Extrait selon l'une quelconque des revendications 1 à 3, **caractérisé en ce que** la solubilité dans l'eau du solvant organique à 20°C est inférieure à 1 % en poids.

5. Extrait selon l'une quelconque des revendications 1 à 4, **caractérisé en ce que** le solvant organique est choisi parmi le cyclohexane, le tétrachlorométhane, le benzène, le n-hexane et le n-pentane.

6. Extrait selon l'une quelconque des revendications 1 à 5, **caractérisé en ce que** le solvant organique est le cyclohexane.

7. Extrait selon l'une quelconque des revendications 1 à 5, **caractérisé en ce que** le solvant organique est le n-pentane.

8. Extrait selon l'une quelconque des revendications 1 à 7, **caractérisé en ce que** l'extrait est dépourvu de constituants colorants.

9. Utilisation d'un extrait selon l'une quelconque des revendications 1 à 8 en tant que principe actif cosmétique.

10. Préparation pour le traitement de la peau humaine ou de cheveux humains, **caractérisée en ce que** la préparation contient un extrait selon l'une quelconque des revendications 1 à 8.

11. Préparation selon la revendication 10, **caractérisée en ce qu'**il s'agit d'un produit de traitement capillaire, en particulier d'un produit de soins capillaires ou de teinture capillaire.

12. Préparation selon la revendication 10, **caractérisée en ce qu'**il s'agit d'un produit de traitement de la peau.

13. Préparation selon l'une quelconque des revendications 10 à 12, **caractérisée en ce qu'**elle contient en outre un agent tensioactif.

14. Préparation selon l'une quelconque des revendications 10 à 13, **caractérisée en ce qu'**elle contient en outre une matière grasse.
